# EUROPÄISCHE PATENTSCHRIFT

(11) **EP 0 916 659 B1**
(45) Veröffentlichungstag und Bekanntmachung des Hinweises auf die Patenterteilung: **29.05.2002**
(21) Anmeldenummer: 98121384.6
(22) Anmeldetag: 10.11.1998
(51) Int. Cl.: C07D 233/64

(54) **Verfahren zur Herstellung von Formylimidazolen**
Process for the preparation of formylimidazoles
Procédé de préparation de formylimidazoles

(30) Priorität: 14.11.1997 CH 263797
(43) Veröffentlichungstag der Anmeldung: 19.05.1999
(73) Patentinhaber: Lonza AG, 3930 Visp (CH)
(72) Erfinder: Heveling, Josef Dr., 3904 Naters (Kanton Wallis) (CH); Wellig, Alain, 3986 Ried-Mörel (Kanton Wallis) (CH)
(74) Vertreter: Winter, Brandl & Partner

(56) Entgegenhaltungen:
- EP-A- 0 514 198
- GB-A- 2 271 353
- US-A- 4 168 964

## Beschreibung

Die vorliegende Erfindung betrifft ein neues Verfahren zur Herstellung von Formylimidazolen der allgemeinen Formel 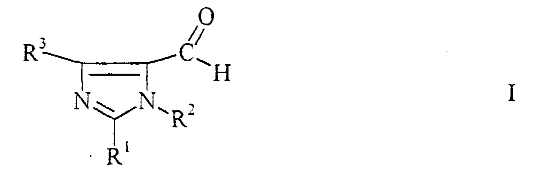 oder 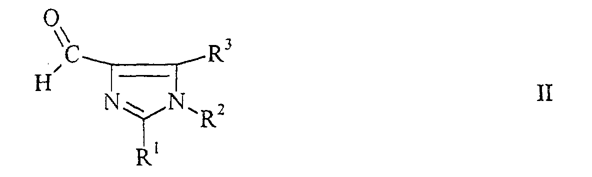 worin R¹ Wasserstoff oder eine gegebenenfalls substituierte Alkylgruppe, R² Wasserstoff, eine gegebenenfalls substituierte Alkyl-, Aryl- oder Arylalkylgruppe und R³ Wasserstoff oder eine gegebenenfalls substituierte Alkylgruppe bedeuten, durch katalytische Oxidation von Hydroxymethylimidazolen der allgemeinen Formel 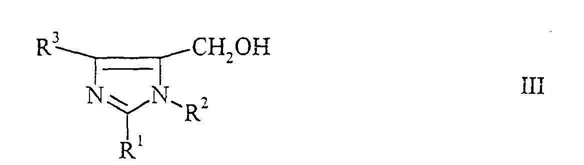 oder 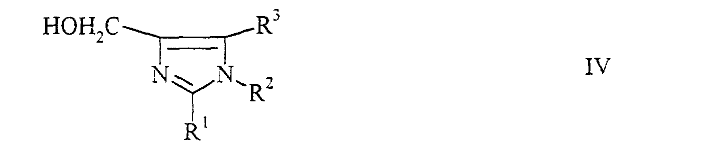 worin R¹, R² und R³ die obengenannte Bedeutung haben.

Formylimidazole sind wichtige Zwischenprodukte z. B. für die Herstellung von pharmazeutischen Wirkstoffen wie Diuretika oder Antihypertonika (WO-A 92/20651). Bisher sind mehrere Verfahren zur Herstellung von Formylimidazolen bekannt.

Die EP-A-0 514 198 und die US-A-4 168 964 beschreiben die Herstellung von Formylimidazolen durch Oxidation von Hydroxymethylimidazolen, wobei als Oxidationsmittel Mangandioxid bzw. konzentrierte Salpetersäure verwendet wird. In der CH-A-685496 wird ein Verfahren beschrieben, bei dem die katalytische Oxidation von Hydroxymethylimidazolen zu Formylimidazolen in Gegenwart von Edelmetallkatalysatoren wie Platinwismuth, Platinschwarz, Platin oder Palladium auf Aktivkohle unter Einblasen von Sauerstoff durchgeführt wird. Nachteilig bei diesem Verfahren sind die langen Reaktionszeiten von mehreren Stunden und die Bildung von Nebenprodukten.

Die Aufgabe der Erfindung war daher, ein ökonomisches Verfahren zur Herstellung von Formylimidazolen zur Verfügung zu stellen, welches die genannten Nachteile nicht aufweist.

Erfindungsgemäss wird diese Aufgabe durch das Verfahren nach Anspruch 1 gelöst. Darin werden Hydroxymethylimidazole der allgemeinen Formel 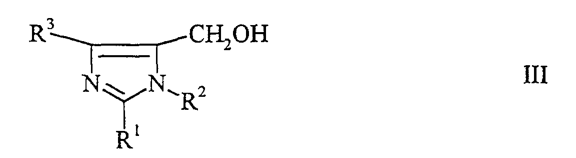 oder 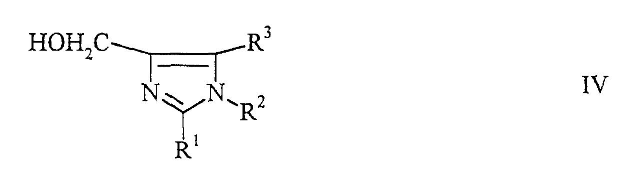 worin R¹, R² und R³ die obengenannte Bedeutung haben, in Gegenwart eines Edelmetallkatalysators und eines Peroxides zu Formylimidazolen der allgemeinen Formel 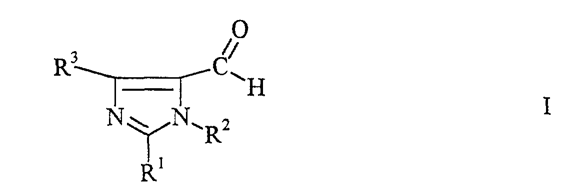 oder 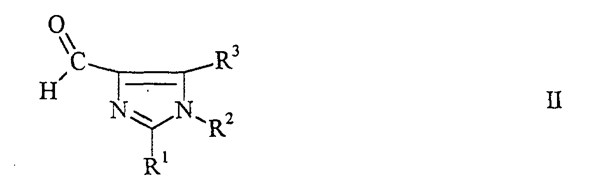 worin R¹, R² und R³ die obengenannte Bedeutung haben, katalytisch oxidiert.

R¹ und R² haben unabhängig voneinander die Bedeutung von Wasserstoff oder einer gegebenenfalls substituierten geradkettigen oder verzweigten C₁₋₆-Alkylgruppe. Namentlich erwähnt seien Methyl, Ethyl, Propyl, Isopropyl, Butyl, Isobutyl, tert-Butyl, Pentyl und seine Isomeren sowie Hexyl und seine Isomeren. R² kann ausserdem gegebenenfalls substituiertes Aryl oder Arylalkyl, insbesondere Phenyl oder Phenylalkyl bedeuten, wobei unter Phenylalkyl vorzugsweise Phenyl-C₁₋₆-alkyl, besonders bevorzugt Benzyl verstanden wird. Substituenten der Alkylgruppen oder der Aromaten der Arylfunktion sind z. B. Halogen, Amino, Alkylamino, Dialkylamino oder Alkoxy, wobei Alkyl wie oben C₁₋₆-Alkyl und Alkoxy C₁₋₆-Alkoxy, beispielsweise Methoxy oder Ethoxy, bedeutet. Unter Halogen ist hier und im folgenden Fluor, Chlor, Brom oder Jod zu verstehen. Besonders bevorzugt hat R¹ die Bedeutung von Butyl und R² die Bedeutung von Wasserstoff.

R³ hat die Bedeutung von Wasserstoff oder einer gegebenenfalls substituierten geradkettigen oder verzweigten C₁₋₆-Alkylgruppe. Namentlich erwähnt seien Methyl, Ethyl, Propyl, Isopropyl, Isobutyl, Butyl, tert-Butyl, Pentyl und seine Isomeren sowie Hexyl und seine Isomeren. Als Substituenten können die oben genannten auftreten. Besonders bevorzugt hat R³ die Bedeutung von Wasserstoff.

Hydroxymethylimidazole als Ausgangsverbindungen können auf einfache Weise z.B. gemäss der Vorschrift nach der WO-A 92/20651 oder gemäss E. F. Godefroi et al., Trav. Chim. Receuil Pays-Bas, 91, 1383 (1972) hergestellt werden.

Als Edelmetallkatalysator kann Platin, Palladium, Rhodium oder Gold eingesetzt werden. Zweckmässig wird das Edelmetall in Kombination mit Metallen wie beispielsweise Wismuth, Blei, Cerium oder Indium als zweiter Komponente eingesetzt. Bevorzugt werden Platin/Wismuth- oder Platin/Blei-Katalysatoren eingesetzt.
Der Edelmetallkatalysator wird als solcher oder gebunden an ein Trägermaterial wie z. B. Aktivkohle, Siliciumdioxid, Aluminiumoxid, Silicium-Aluminiumoxid, Zirkonoxid oder Titanoxid eingesetzt. Bevorzugt wird er gebunden an Aktivkohle eingesetzt. Edelmetallkatalysatoren, welche an Aktivkohle gebunden sind, sind im Handel, z. B. von Degussa, erhältlich.
Der Anteil des an ein Trägermaterial gebundenen Edelmetalles beträgt zweckmässig zwischen 0,1 und 15 Gewichts-%, vorzugsweise zwischen 0,5 und 7 Gewichts-%, bezogen auf das Trägermaterial.
Der Edelmetallkatalysator wird vorzugsweise in einer Menge von 0,05 bis 1,0 Mol-% auf Edelmetallbasis, bezogen auf das Hydroxymethylimidazol, besonders bevorzugt in einer Menge von 0,1 bis 0,4 Mol-% auf Edelmetallbasis, bezogen auf das Hydroxymethylimidazol, eingesetzt.
Als Peroxide werden organische oder anorganische Peroxide eingesetzt. Gut geeignet sind beispielsweise Wasserstoffperoxid, Perborate, eine Percarbonsäure, tert-Butylhydroperoxid, Cumolhydroperoxid, Perbenzoesäure, m-Chlorperbenzoesäure, Monoperphthalsäure oder Peressigsäure. Besonders gut geeignet ist Wasserstoffperoxid, welches zweckmässig als 10 - 30 %-ige wässerige Lösung eingesetzt wird.

Zweckmässig erfolgt die katalytische Oxidation in Gegenwart von Wasser, einem mit Wasser mischbaren Lösungsmittel, einem mit Wasser nicht mischbaren organischen Lösungsmittel oder Mischungen davon, in alkalischem Milieu.

Gut geeignete mit Wasser mischbare Lösungsmittel sind beispielsweise Alkohole oder Carbonsäuren mit 1 bis 6 C-Atomen oder Ketone, wie beispielsweise Aceton oder Methylethylketon. Geeignete mit Wasser nicht mischbare organische Lösungsmittel sind z. B. Isobutylmethylketon oder Essigsäureethylester. Vorzugsweise wird Wasser eingesetzt.

Es hat sich als vorteilhaft erwiesen, wenn das alkalische Milieu durch Zugabe von einem Alkalihydroxid, einem Alkalicarbonat oder einem Alkaliacetat erhalten wird. Alkalihydroxid wird vorzugsweise im Verhältnis 1 : 0,05 bis 1,2, vorzugsweise 1 : 0,1 bis 1, bezogen auf die eingesetzte Molmenge des Hydroxymethylimidazols der allgemeinen Formel III oder IV eingesetzt.

Die katalytische Oxidation erfolgt zweckmässig bei einer Temperatur von 20 - 120 °C, vorteilhaft bei 50 - 80°C.
Nach einer üblichen Dosierungszeit des Peroxids von ungefähr 1 Stunde kann, nach genügender Nachreaktionszeit, die Verbindung der allgemeinen Formel I oder II auf fachmännisch übliche Weise isoliert werden

Die Isolierung des Produktes erfolgt zweckmässig, abhängig vom Lösungsmittelsystem, entweder durch Kristallisation und Filtration oder durch Extraktion mit einem geeigneten Lösungsmittel.
Der verwendete Katalysator kann ohne Aktivitätsverlust mehrmals verwendet werden.

### Beispiele:

### Beispiel 1

### Herstellung von 2-n-Butyl-5-formylimidazol

1,5 g 2-n-Butyl-5-hydroxymethylimidazol, 1,5 g Dodecan als interner GC-Standard, 0,3 g 5% Platin und 5% Wismuth auf Aktivkohle (61,3% Wasser enthaltend), 20 g Isobutylmethylketon und 2,4 g 1,6%-ige NaOH-Lösung wurden vorgelegt und unter Rühren auf ca. 58 °C aufgeheizt. Bei 58 - 64 °C wurden in 45 min 2,9 g 15,7%-ige wässerige H₂O₂-Lösung zugetropft. Man liess 15 min nachreagieren, dann wurde das Reaktionsgemisch abfiltriert. Das Filtrat wurde in einen Scheidetrichter überführt, die H₂O-Phase abgetrennt, die org. Phase aufkonzentriert, gekühlt, das Produkt auskristallisiert und abfiltriert.
Die Ausbeute wurde mittels GC (interner Standard) bestimmt.
Reaktionsausbeute: 88,2% (6,75% Edukt).

### Beispiel 2

### Herstellung von 2-n-Butyl-5-formylimidazol

2,0 g 2-n-Butyl-5-hydroxymethylimidazol, 0,3 g 5% Platin und 5% Wismuth auf Aktivkohle (61,3% Wasser enthaltend), 13 ml 1N NaOH-Lösung und 7 g H₂O wurden vorgelegt und unter Rühren auf ca. 60 °C aufgeheizt. Bei 60 - 64 °C wurden in 45 min 3,4 g 15%-ige wässerige H₂O₂-Lösung zugetropft. Man liess 15 min nachreagieren, dann wurde das Reaktionsgemisch abfiltriert. Das Filtrat stellte man mit 20 % H₂SO₄ von pH 13,4 auf pH 9,0, dabei entstand eine hellgelbe Suspension. Diese wurde abgekühlt, und man konnte das Rohprodukt abfiltrieren und/oder mit Methylenchlorid extrahieren.
Die Reaktion wurde mit GC-Analyse überwacht.
Reaktionsausbeute: 100% (0% Edukt).

### Beispiel 3

### Herstellung von 2-n-Butyl-5-formylimidazol

Es wurde verfahren wie in Beispiel 2 beschrieben, jedoch wurden anstelle von 0,3 g 5% Platin und 5% Wismuth auf Aktivkohle 0,3g 5% Platin und 5% Blei auf Aktivkohle (55,7% Wasser enthaltend) eingesetzt.
Reaktionsausbeute: 99,5 (0,5% Nebenprodukte).

### Beispiel 4

### Herstellung von 2-n-Butyl-5-formylimidazol

4,0 g 2-n-Butyl-5-hydroxymethylimidazol, 0,6 g 5% Platin und 5% Wismuth auf Aktivkohle (61,3% Wasser enthaltend), und 25,6 ml 1N NaOH-Lösung wurden vorgelegt und unter Rühren auf ca. 60 °C aufgeheizt. Bei 60 - 64 °C wurden in 45 min 6,8 g 15%-ige wässerige H₂O₂-Lösung zugetropft. Man liess 15 min nachreagieren, dann wurde das Reaktionsgemisch abfiltriert. Das Filtrat stellte man mit 20% H₂SO₄ von pH 13,2 auf pH 9,0, dabei entstand eine gelbe Suspension. Diese wurde abgekühlt, und man konnte das Rohprodukt abfiltrieren und/oder mit Methylenchlorid extrahieren.
Die Reaktion wurde mit GC-Analyse überwacht.
Reaktionsausbeute: 98,2% (1,8% Edukt).

### Beispiel 5

### Herstellung von 2-n-Butyl-5-formylimidazol

Es wurde verfahren wie in Beispiel 4 beschrieben, jedoch wurde anstelle bei 60 - 64 °C bei 50 - 54 °C 15%-ige H₂O₂-Lösung zugetropft.
Reaktionsausbeute: 97,6% (2,4% Edukt).

### Beispiel 6

### Herstellung von 2-n-Butyl-5-formylimidazol

Es wurde verfahren wie in Beispiel 4 beschrieben, jedoch wurde anstelle bei 60 - 64 °C bei 70 - 74 °C 15%-ige H₂O₂-Lösung zugetropft.
Reaktionsausbeute: 97.7% (2,1% Edukt).

### Beispiel 7

### Herstellung von 2-n-Butyl-5-formylimidazol ausgehend vom Rohedukt

4,4g 2-n-Butyl-5-hydroxymethylimidazol (Rohedukt 90,4%), 0,6 g 5% Platin und 5% Wismuth auf Aktivkohle (61,3% Wasser enthaltend), 25,6 ml 1N NaOH-Lösung und 5 ml Methanol wurden vorgelegt und unter Rühren auf ca. 60 °C aufgeheizt. Bei 60 - 64 °C wurden in 45 min 6,8 g 15%-ige wässerige H₂O₂-Lösung zugetropft. Man liess 15 min nachreagieren, dann wurde das Reaktionsgemisch abfiltriert. Das Filtrat stellte man mit 20% H₂SO₄ von pH 13,0 auf pH 9,0, dabei entstand eine gelbe Suspension. Diese wurde abgekühlt, und man konnte das Rohprodukt abfiltrieren und/oder mit Methylenchlorid extrahieren.
Die Reaktion wurde mit GC-Analyse überwacht.
Reaktionsausbeute: 94,5% (3,5% Edukt).

### Beispiel 8 (Vergleichsversuch nach CH-A 685 496 mit Luft als Oxidationsmittel)

### Herstellung von 2-n-Butyl-5-formylimidazol

4,6 g 2-n-Butyl-5-hydroxymethylimidazol, 4,6 g Dodecan als interner GC-Standard, 0,6 g 5% Platin und 5% Wismuth auf Aktivkohle (61,3% Wasser enthaltend), 42 g Isobutylmethylketon und 7,5 g 1,6%-ige NaOH-Lösung wurden vorgelegt und unter Rühren auf 80 °C aufgeheizt. Bei 80 °C wurden 3,6 Normliter Luft/h in die Lösung eingeleitet bis die Sauerstoffaufnahme beendet war (350 min!). Das Reaktionsgemisch wurde abfiltriert. Das Filtrat gab man in einen Scheidetrichter, die H₂O-Phase wurde abgetrennt, die org. Phase aufkonzentriert, gekühlt, das Produkt auskristallisiert und abfiltriert.
Die Reaktion wurde mit GC-Analyse (interner Standard) überwacht.
Reaktionsausbeute: 90,0% (0,3% Edukt).

### Beispiel 9 (Katalysatorrückführung)

20,9 g 2-n-Butyl-5-hydroxymethylimidazol (Rohedukt 95,3%), 3,2 g 5% Platin und 5% Blei auf Aktivkohle (55,7% Wasser enthaltend), 130 ml 1N NaOH-Lösung und 22 ml Methanol wurden vorgelegt und unter Rühren auf ca. 60 °C aufgeheizt. Bei 60 °C wurden in 60 min 22,5 g 20%-ige wässerige H₂O₂-Lösung zugetropft.
Man liess 10 min nachreagieren; dann wurde das Reaktionsgemisch abfiltriert. Das Filtrat stellte man mit 50% H₂SO₄ von pH 12,8 auf pH 7,5, dabei entstand eine gelbliche Suspension.
Das Methanol und etwas Wasser wurden abdestilliert, die Suspension wurde auf 2 °C gekühlt und das Produkt abfiltriert. Das Produkt wurde bei 65 °C und 30 mbar getrocknet. Nach dem ersten Einsatz des Katalysators wurden 18 g hellgelbe Substanz isoliert (Gehalt: 98,5%, HPLC Gew.-%). Die isolierte Ausbeute betrug 90%.
Die Wiedereinsetzbarkeit des Katalysators wurde mittels GC-Analyse (Norm-%) überwacht. Der Katalysator wurde insgesamt achtmal eingesetzt. Katalysatorverluste wurden dabei nicht ersetzt.

| **Einsatz Katalyt** | **2-n-Butyl-5-formylimidazol (GC Norm-%]** | **nicht-umgesetztes 2-n-Butyl-5- hydroxymethylimidazol [GC Norm-%]** |
|---|---|---|
| 1 | 97,9 | 1 |
| 2 | 97,5 | 1,3 |
| 3 | 97,0 | 1,9 |
| 4 | 97,5 | 1,7 |
| 5 | 98,0 | 0 |
| 6 | 97,2 | 1,7 |
| 7 | 95,9 | 2,9 |
| 8 | 96,6 | 1,9 |

## Patentansprüche

1. Verfahren zur Herstellung von Formylimidazolen der allgemeinen Formel 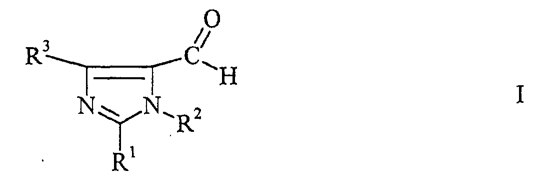 oder 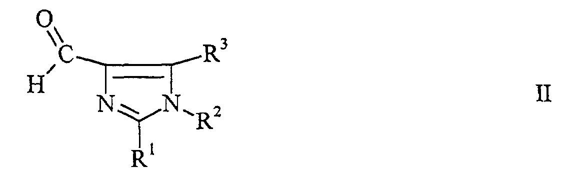 worin R¹ Wasserstoff oder eine gegebenenfalls mit Halogen, Amino, C₁₋₆-Alkylamino, Di-C₁₋₆-alkylamino oder C₁₋₆-Alkoxy substituierte C₁₋₆-Alkylgruppe, R² Wasserstoff, eine gegebenenfalls wie oben substituierte C₁₋₆-Alkyl-, Aryl- oder Aryl-C₁₋₆--alkylgruppe und R³ Wasserstoff oder eine gegebenenfalls wie oben substituierte C₁₋₆-Alkylgruppe bedeuten, durch katalytische Oxidation von Hydroxymethylimidazolen der allgemeinen Formel 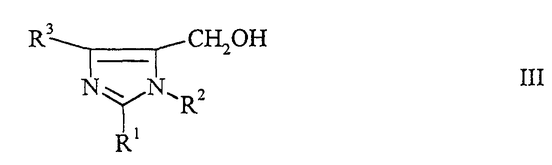 oder 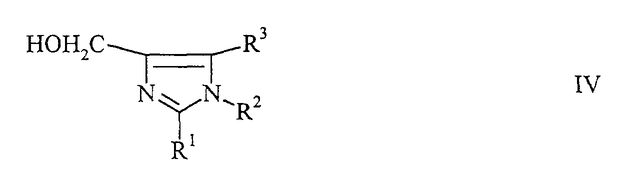 worin R¹, R² und R³ die obengenannte Bedeutung haben, in Gegenwart eines Edelmetallkatalysators, **dadurch gekennzeichnet, dass** die katalytische Oxidation in Gegenwart eines Peroxides erfolgt.

2. Verfahren nach Anspruch 1, **dadurch gekennzeichnet, dass** R¹ eine Butylgruppe ist.

3. Verfahren nach Anspruch 1 oder 2, **dadurch gekennzeichnet, dass** R² und R³ Wasserstoff sind.

4. Verfahren nach einem der Ansprüche 1 bis 3, **dadurch gekennzeichnet, dass** der Edelmetallkatalysator ein Platin/Wismuth-Katalysator oder ein Platin/Blei-Katalysator ist.

5. Verfahren nach einem der Ansprüche 1 bis 4, **dadurch gekennzeichnet, dass** das Peroxid Wasserstoffperoxid ist.

6. Verfahren nach einem der Ansprüche 1 bis 5, **dadurch gekennzeichnet, dass** die katalytische Oxidation in Gegenwart von Wasser, einem mit Wasser mischbaren Lösungsmittel, einem mit Wasser nicht mischbaren organischen Lösungsmittel oder Mischungen davon, in alkalischem Milieu durchgeführt wird.

7. Verfahren nach Anspruch 6, **dadurch gekennzeichnet, dass** das alkalische Milieu durch Zugabe eines Alkalihydroxids, Alkalicarbonats oder Alkaliacetats zum Reaktionsgemisch erhalten wird.

8. Verfahren nach einem der Ansprüche 1 bis 7, **dadurch gekennzeichnet, dass** die Umsetzung bei einer Temperatur von 20 - 120 °C durchgeführt wird.

## Claims

1. Process for the preparation of formylimidazoles corresponding to the general formula 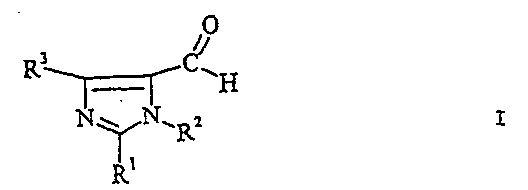 or 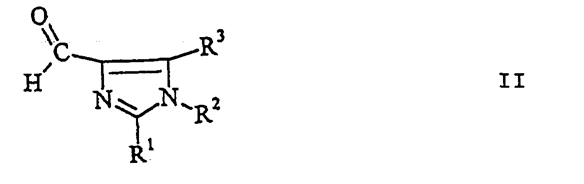 wherein R¹ denotes hydrogen or a C₁₋₆-alkyl group optionally substituted with-halogen, amino, C₁₋₆-alkylamino, di-C₁₋₆-alkylamino or C₁₋₆-alkoxy, R² denotes hydrogen, a C₁₋₆-alkyl, aryl or aryl-C₁₋₆-alkyl group substituted as above and R³ denotes hydrogen or a C₁₋₆-alkyl group optionally substituted as above,
by catalytic oxidation of hydroxymethylimidazoles corresponding to the general formula 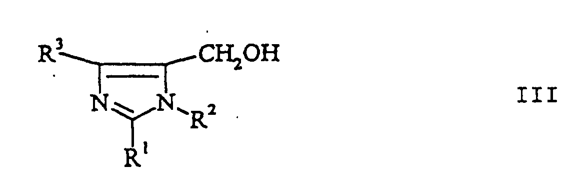 or 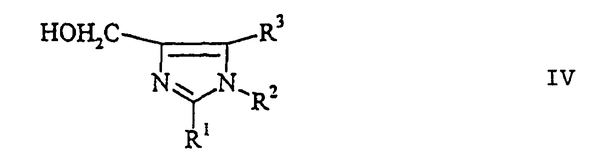 wherein R¹, R² and R³ have the meanings given above, in the presence of a precious metal catalyst, **characterised in that** the catalytic oxidation is carried out in the presence of a peroxide.

2. Process according to claim 1, **characterised in that** R¹ is a butyl group.

3. Process according to claim 1 or 2, **characterised in that** R² and R³ are hydrogen.

4. Process according to one of claims 1 to 3, **characterised in that** the precious metal catalyst is a platinum/bismuth catalyst or a platinum/lead catalyst.

5. Process according to one of claims 1 to 4, **characterised in that** the peroxide is hydrogen peroxide.

6. Process according to one of claims 1 to 5, **characterised in that** the catalytic oxidation is carried out in alkaline medium in the presence of water, of a solvent which is miscible with water, of an organic solvent which is immiscible with water or mixtures of these.

7. Process according to claim 6, **characterised in that** the alkaline medium is obtained by the addition of an alkali hydroxide, of an alkali carbonate or of an alkali acetate to the reaction mixture.

8. Process according to one of claims 1 to 7, **characterised in that** the reaction is carried out at a temperature of 20°C to 120°C.

## Revendications

1. Procédé de préparation de formylimidazoles de formule générale 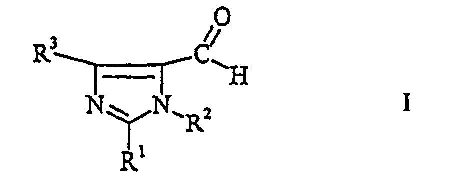 ou 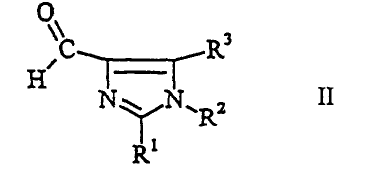 dans lesquelles R¹ représente un atome d'hydrogène ou un groupe alkyle en C₁-C₆ éventuellement substitué par halogéno, amino, alkylamino en C₁-C₆, di(alkyl en C₁-C₆)amino ou alcoxy en C₁-C₆, R² est un atome d'hydrogène, un groupe alkyle en C₁-C₆, aryle ou aryl(alkyle en C₁-C₆) éventuellement substitué comme ci-dessus, et R³ représente un atome d'hydrogène ou un groupe alkyle en C₁-C₆ éventuellement substitué comme ci-dessus, par oxydation catalytique d'hydroxyméthylimidazoles de formule générale 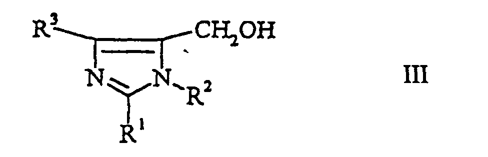 ou 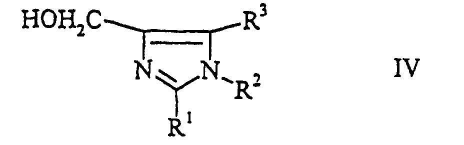 dans lesquelles R¹, R² et R³ ont la signification indiquée ci-dessus, en présence d'un catalyseur à base d'un métal noble, **caractérisé en ce que** l'oxydation catalytique s'effectue en présence d'un peroxyde.

2. Procédé selon la revendication 1, **caractérisé en ce que** R¹ est un groupe butyle.

3. Procédé selon la revendication 1 ou 2, **caractérisé en ce que** R² et R³ sont des atomes d'hydrogène.

4. Procédé selon l'une des revendications 1 à 3, **caractérisé en ce que** le catalyseur à base de métal noble est un catalyseur à base de platine/bismuth ou un catalyseur à base de platine/plomb.

5. Procédé selon l'une des revendications 1 à 4, **caractérisé en ce que** le peroxyde est le peroxyde d'hydrogène.

6. Procédé selon l'une des revendications 1 à 5, **caractérisé en ce que** l'oxydation catalytique s'effectue en milieu alcalin en présence d'eau, d'un solvant miscible à l'eau, d'un solvant organique non miscible à l'eau ou d'un de leurs mélanges.

7. Procédé selon la revendication 6, **caractérisé en ce que** le milieu alcalin est obtenu par addition au mélange réactionnel d'un hydroxyde de métal alcalin, d'un carbonate de métal alcalin ou d'un acétate de métal alcalin.

8. Procédé selon l'une des revendications 1 à 7, **caractérisé en ce que** la réaction s'effectue à une température de 20 à 120°C.
